# EUROPEAN PATENT SPECIFICATION

(11) **EP 1 926 473 B1**
(45) Date of publication and mention of the grant of the patent: **18.12.2013**
(21) Application number: 06806766.9
(22) Date of filing: 12.09.2006
(51) Int. Cl.: A61K 9/00, A61K 9/127, A61K 31/4409

(54) **ANTIMICROBIAL PREPARATIONS HAVING A CONTENT OF OCTENIDINE DIHYDROCHLORIDE ENCAPSULATED IN LIPOSOMES**
ANTIMIKROBIELLE ZUBEREITUNGEN MIT EINEM IN LIPOSOMEN VERKAPSELTEN GEHALT AN OCTENIDINDIHYDROCHLORID
PREPARATIONS ANTIMICROBIENNES COMPRENANT DU DICHLORHYDRATE D'OCTENIDINE ENCAPSULE DANS DES LIPOSOMES

(30) Priority: 15.09.2005 DE 102005045146
(43) Date of publication of application: 04.06.2008
(60) Divisional application: 12179218.8
(73) Proprietor: AIR LIQUIDE SANTE (INTERNATIONAL), 75007 Paris (FR); Schülke & Mayr GmbH, 22851 Norderstedt (DE)
(72) Inventor: BEHRENDS, Sabine, 25421 Appen (DE); SIEBERT, Jörg, 22848 Norderstedt (DE); GOLOMBIEWSKI, Mona, 21335 Lüneburg (DE); KRAMER, Axel, 17489 Greifswald (DE); MÜLLER, Gerald, 17498 Hinrichshagen (DE)
(74) Representative: Conan, Philippe Claude
(86) International application number: PCT/EP2006/066282
(87) International publication number: WO 2007/031519

(56) References cited:
- WO-A-2006/099359
- DE-A1- 2 708 331
- US-A1- 2003 068 365
- SEDLOCK D M ET AL: "Microbicidal activity of octenidine hydrochloride, a new alkanediylbis[pyridine] germicidal agent." ANTIMICROBIAL AGENTS AND CHEMOTHERAPY DEC 1985, vol. 28, no. 6, December 1985 (1985-12), pages 786-790, XP002426771 ISSN: 0066-4804
- REIKE H: "[Wound healing and local wound treatment in diabetic foot syndrome]" DER INTERNIST OCT 1999, vol. 40, no. 10, October 1999 (1999-10), pages 1009-1017, XP002426867 ISSN: 0020-9554
- FRIESE K ET AL: "Topical antiseptics as an alternative in the treatment of acute vulvovaginal candidosis." ARCHIVES OF GYNECOLOGY AND OBSTETRICS, vol. 268, no. 3, August 2003 (2003-08), pages 194-197, XP002426772 ISSN: 0932-0067
- LANGER S ET AL: "THE IMPACT OF TOPICAL ANTISEPTICS ON SKIN MICROCIRCULATION" EUROPEAN JOURNAL OF MEDICAL RESEARCH, MUNICH, DE, vol. 9, no. 9, 29 September 2004 (2004-09-29), pages 449-454, XP008058339 ISSN: 0949-2321

## Description

The invention relates to the use of liposomes comprising phospholipide for manufacturing an antimicrobial preparations which comprise octenidine dihydrochloride in liposomes. The invention additionally relates to a process for manufacturing the preparations. The antimicrobial active ingredient octenidine dihydrochloride is a bispyridiniumalkane having the formula: 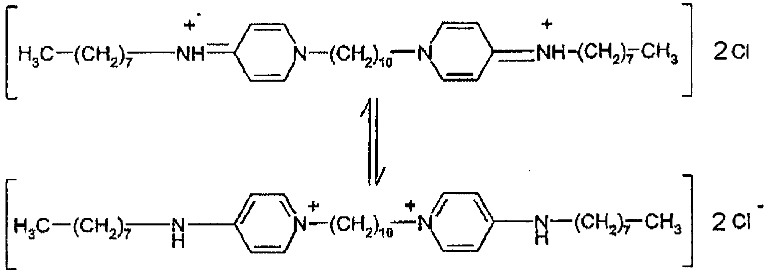 and has been successful for many years as mucosal and wound antiseptic in the commercial product Octenisept^{®}. Octenisept^{®} comprises besides octenidine dihydrochloride and phenoxyethanol also glycerol, sodium D-gluconate and cocamidopropyldimethylammonium acetate in aqueous solution.

The bacteriostatic activity and dental plaque-preventing activity of octenidine dihydrochloride is described in DE 27 08 331 C2. EP 0 411 315 A1 discloses an aqueous antiseptic composition which is suitable in particular as mucosal antiseptic and for wound treatment and comprises octenidine dihydrochloride plus phenoxyethanol and/or phenoxypropanol. DE 102 05 883 A1 relates to an aqueous antiseptic which can be adjusted to isotonicity and comprises octenidine dihydrochloride.

The excellent antimicrobial activity of octenidine dihydrochloride against a large number of microbes is demonstrated in numerous studies. A reduction factor of 5 decadic units is achieved even with concentrations of > 0.001% octenidine dihydrochloride (i.e. 1/100 of the active ingredient concentration in Octenisept^{®}) within 1 min. Although the cytotoxicity of the active ingredient in the concentrations necessary for activity is low, it is present even at concentrations higher than 0.001% by weight octenidine dihydrochloride. Hence there are limits on the use of octenidine dihydrochloride for example on chronic wounds.

It was thus an object of this invention to develop preparations based on octenidine dihydrochloride which - while retaining the activity - have distinctly better tolerability. It was specifically intended to achieve an improvement in the therapeutic range, a reduction in the risk of local side effects and a distinctly improved acceptance by the user.

It has now surprisingly emerged that the object is achieved by the use of liposomes comprising phospholipids for manufacturing an antimicrobial preparation which comprises octenidine dihydrochloride encapsulated in liposomes, for reducing the cytotoxicity of the preparation.

The invention is based inter alia on the finding that an unchanged reduction in microbes - by comparison with an aqueous octenidine dihydrochloride solution - is achieved through interactions of octenidine dihydrochloride with for example phospholipid liposomes, whereas the cytotoxicity is greatly diminished by comparison with the solution. Hence, octenidine dihydrochloride bound on or in liposomes results in very well tolerated preparations according to the invention.

### Preparation

Preferred preparations according to the invention are in the form of solution, dispersion, cream (O/W, W/O, 0/W/O, W/O/W or ambiphilic cream), ointment or suppository. The content of octenidine dihydrochloride is preferably from 0.001 to 5% by weight, more preferably from 0.003 to 1% by weight, in particular from 0.005 to 0.1% by weight, such as, for example, 0.01 to 0.06% by weight, in each case based on the complete preparation. Besides the octenidine dihydrochloride encapsulated in the liposomes, the preparation according to the invention may comprise further octenidine dihydrochloride which, for example, is adsorbed on the liposomes. The amount of octenidine dihydrochloride encapsulated in liposomes is preferably at least 20% by weight, more preferably at least 30% by weight, in particular at least 50% by weight, i.e. 70% by weight, based on the total amount of octenidine dihydrochloride present in the preparation.

It is possible according to the invention for the preparation where appropriate to comprise further active ingredients which supplement the activity of octenidine dihydrochloride and can, if they are combined with octenidine dihydrochloride, be employed in a distinctly lower concentration than in known commercial products.

Suitable dosage forms are semisolid:
Ointments (lat. unguenta) are spreadable preparations which are intended for use on the skin by application or rubbing in. They consist of one or more ointment bases (such as petrolatum, wool fat, lanolin etc.) into which the active ingredient is incorporated. The active ingredient should be dissolved or very finely dispersed. In order to increase the solubility, ointments often comprise water or oils. However, the fat/oil content in an ointment is higher than the water content.
Creams are very similar to ointments but the water content therein is higher than the fat/oil content.
CreSa is a short designation for a combination of cream and ointment.
   The viscosity of ointments and creams manufactured in the invention is generally from 500 to 15 000 mPa•s, preferably 1000 to 1 0 000 mPa•s, measured with a rotational viscometer at 95 ^{s-1} and 20°C (e.g. of the type RV20, System M5, measuring unit SV1, from Thermo Haake).
Paste is the designation for ointments in which ingredients in powder form (e.g. zinc oxide, talc etc.) are dispersed in large amount. Pastes comprise no water and, through the large proportion of powders, are substantially firmer than ointments.
Hydroalcoholic gels (hydrogels) are valued for their transparency and non-greasy characteristics. Lipophilic gels (oleogels) are likewise employed because of their aesthetic appearance and their consistency-conferring properties. Gels are predominantly intended for external use and should be applied thinly.
A hydrogel is a usually translucent composition which is manufactured with the aid of gelatin, tragacanth, Carbopol or similar swelling agents with the addition of water and glycerol. They have a cooling effect through the evaporation of the water.
Lipophilic gels include a lipophilic phase. Matrix formers employed, besides higher-molecular weight homologues of the lipid phase, are also organo-modified bentonites (Benton^{®}) and colloidal silicon dioxide.
Emulsion means preparations consisting of immiscible liquids, e.g. oil and water. A distinction is made between W/O (water in oil) or O/W (oil in water) emulsions and ambiphilic emulsions. The latter must be vigorously shaken before use. Addition of an emulsifier makes it possible for the liquids to be distributed extremely finely within one another, and the emulsions are thus stable, i.e. the oil and the water do not separate again. Depending on the mode of application, emulsions are intended for internal or external use. Emulsions for external use are frequently referred to as lotions. This takes the form of an oil-in-water emulsion.
CreLo means a combination of cream and lotion.
Suppositories (lat. suppositoria) are single-dose pharmaceutical preparations which have various shapes and are intended for introduction into the rectum and there deliver the active ingredient after melting or dissolving. They consist of a hard fat (e.g. Stadimol) or polyethylene glycol, in which the active ingredient is incorporated with input of heat. This heated composition is then poured into moulds. Hard fat suppositories are heat-sensitive and should therefore never be stored above 25°C. A normal suppository size for adults is about 2 g and for children about 1 g. Suppositories should best be introduced after defecation and in supine position. Introduction can be facilitated by previously dipping the suppository in water. Rubbing in creams or ointments should be avoided because it may impair the activity of the suppositories.

Suppositories to be introduced into the vagina are differentiated into vaginal suppositories and vaginal pessaries (lat. ovulum). The vaginal suppositories are similar in manufacture and basic composition to the "normal" suppositories. Vaginal pessaries mostly consist of gelatin, water and glycerol and have a spherical shape. The weight of both dosage forms is about 3 g• Application should take place where possible in the evening and in the supine position. In this case too, creams should be dispensed with for the introduction. An introduction aid is supplied by the manufacturer together with various suppositories. Storage below 25°C is important here too.

Examples of dosage forms are (data in % by weight):

### I. Cream

| | |
|---|---|
| 3 to 20% | fat content, preferably 3 to 20% medium-chain triglycerides |
| 0.5 to 10% | humectants such as glycerol, propylene glycol, preferably 0.5 to 10% glycerol, |
| 1 to 10% | emulsifier, preferably 1 to 10% glycerol monoester, glycerol diester, particularly preferably 1 to 10% glycerol monostearate, |

octenidine dihydrochloride
phosphatidylcholine with acyl, radicals derived from saturated fatty acids;

### II. O/W cream

| | |
|---|---|
| 5 to 20% | fat content, preferably 5 to 20% octyldodecanol |
| 0.5 to 10% | humectants such as glycerol, propylene glycol, 1,2-pentanediol (pentylene glycol), particularly preferably 0.5 to 10% pentylene glycol, |

octenidine dihydrochloride,
phosphatidylcholine with acyl radicals derived from saturated fatty acids;

### III. Hydrogel

| | |
|---|---|
| 0.5 to 10% | humectants such as glycerol, propylene glycol, particularly preferably 0.5 to 10% propylene glycol, |
| 0.05 to 2% | thickeners, preferably 0.05 to 2% carboxylates, particularly preferably 0.05 to 2% sodium carboxyvinyl polymer, |

octenidine dihydrochloride,
phosphatidylcholine with acyl radicals derived from saturated fatty acids;

### IV. Mixed micelles

| | |
|---|---|
| 5 to 30% | nonionic emulsifier, preferably 5 to 30% sorbitan ester, particularly preferably 5 to 30% polyoxyethylene sorbitan monolaurate such as, for example, polysorbate 20 |

octenidine dihydrochloride,
phosphatidylcholine with acyl radicals derived from fatty acids,

The preparations manufactured in the invention can be employed for example for the following indications:

### 1. Wound treatment

In a first embodiment, the preparation manufactured in the invention is employed for the treatment of wounds. This entails preferably choosing an emulsifier-free formulation which comprises a large extent of moisture factors (e.g. a gel).

### 2. Atopic dermatitides and infected eczemas

A formulation of a preparation comprising octenidine dihydrochloride for use according to the invention for the treatment of infected eczemas is preferably, according to skin type, an oil-in-water (O/W), water-in-oil (W/O) or ambiphilic emulsion (creams).

### 3. Dermatomycoses

Gels and creams are preferably used as formulation for the semisolid preparation for the treatment of mycoses.

### 4. Vaginal infections

Dosage forms suitable and preferred for the treatment of vaginal infections are creams and suppositories. Octenidine dihydrochloride in preparations of these types displays a particularly advantageous effect because it is active both against fungi and against bacteria, and the use of two different products is unnecessary.

### Liposomes

Liposomes are spherical structures (diameter 25 nm to 1 µm) composed of one or more concentric lipid bilayers with aqueous interior (lipid vesicle). Vesicles of this type can be manufactured by very fine mechanical dispersion for example of phospholipids such as phosphatidylcholine (lecithin) in aqueous media. A preferred diameter of the liposomes in this connection is from 50 to 400 nm.

The amount of liposome-forming substance, in particular phosphatidylcholine, is preferably in the range from 0.1 to 30% by weight, preferably 0.5 to 20% by weight, in particular 1 to 10% by weight, for example 3 to 8% by weight, based on the complete preparation. Preparations preferred in this connection are those where the liposomes are formed from (glycerol) phospholipid, preferably phosphatidylcholine (lecithin). The acyl radicals of the phosphatidylcholine may be derived from saturated or unsaturated fatty acids.

The preparations manufactured in the invention can be manufactured by processes known in the state of the art. Liposomes typically form spontaneously at high shear from an aqueous solution, for example of the phospholipids, above the transition temperature. The procedure in this connection may be, as shown in the examples, to mix water (or an aqueous solution of another ingredient of the preparation) at elevated temperature (for example about 70°C) with phospholipid and octenidine dihydrochloride, and then to homogenize this mixture.

In a further embodiment, the invention thus relates to such a process.

The advantages of the invention are evident in particular from the following examples. All percentage data therein are based on weight.

### Examples

### Materials used

1,2-Diacyl-*sn*-glycero-3-phosphatidylcholines (lecithins)

R¹-O-CH (CH₂OR²) CH₂-O-P (O) (O) -O-CH₂-CH₂-N(CH₃)₃

### Phospholipon^{®} 90 G

R¹, R² = acyl radicals of fatty acids

| | |
|---|---|
| average molecular formula | C₄₃H₈₇NO₈P |
| average molecular weight | 775.5 g/mol |

### Phospholipon^{®} 90 H

R¹, R² = acyl radicals of saturated fatty acids

| | |
|---|---|
| average molecular formula | C₄₃H₉₅NO₈P |
| average molecular weight | 784.6 g/mol |

### Preparation "octenidine monoproduct"

| | |
|---|---|
| octenidine dihydrochloride | 0.10% |
| glycerol 85% | 2.85% |
| water | 97.05% |

### Preparation 1 according to the invention (dispersion)

| | |
|---|---|
| octenidine dihydrochloride | 0.05% |
| Phospholipon^{®} 90 H | 6.00% |
| water | 93.95% |

The two further concentrations (0.025% and 0.0125%) were prepared by dilution with water.

### Example 1: Retention of activity (quantitative suspension test)

### Method

The bactericidal and fungicidal activity was determined in the quantitative suspension test with high protein loading ("dirty conditions") as specified in the standard methods of the Deutsche Gesellschaft für Hygiene and Mikrobiologie e.V. for testing chemical disinfection methods (date: 1 September 2001). For methodological reasons, products ready for use can be tested only in concentrations of ≤ 80%.

### Test organisms

| | |
|---|---|
| Staphylococcus aureus | ATCC 6538 |
| Escherichia coli | ATCC 10538 |

### Results:

| **Test product** | **Octenidine dihydrochloride concentration** | **1g reduction factor** | | | |
|---|---|---|---|---|---|
| | | **E. coli** | | **S. aureus** | |
| | | **30 min** | **60 min** | **30 min** | **60 min** |
| **Octenidine monoproduct** | 0.05% | > 6 | > 6 | > 6 | > 6 |
| | 0.025% | > 6 | > 6 | > 6 | > 6 |
| | 0.0125% | > 6 | > 6 | > 6 | > 6 |
| **Preparation 1** | 0.05% | > 6 | > 6 | > 6 | > 6 |
| | 0.025% | > 6 | > 6 | > 6 | > 6 |
| | 0.0125% | > 6 | > 6 | > 6 | > 6 |

### Example 2: Further preparations according to the invention with liposomal octenidine hydrochloride

### I. Cream with Phospholipon^{®} 90 H and octenidine dihydrochloride

| | |
|---|---|
| 6.00% | Phospholipon^{®} 90 H |
| 0.05% | octenidine dihydrochloride |
| 10.00% | medium-chain triglycerides, at least 95% saturated fatty acids with 8 to 10 carbon atoms (Miglyol 810) |
| 5.00% | glycerol 100% |
| 0.50% | **α**-DL-tocopherol |
| 5.00% | glycerol monostearate |
| 70.45% | water, demin. |

a. Heat water to 70°C.
   Add Phospholipon^{®} 90 H and octenidine dihydrochloride and stir in.
   Then homogenize at the highest setting for 30 min.
b. Mix.Miglyol 810, glycerol, tocopherol and glycerol monostearate and heat to 40°C.
   Add fat phase, homogenizing with an intermediate speed.
   Then emulsify at the highest setting for 2 min. Stir cream until cold while at intervals homogenizing briefly at intermediate speed.

This formulation was prepared in an IKA L 1000 laboratory reactor with anchor stirrer (= 100 rpm) and Ultra-Turrax T25 (13 500-24 000 rpm).

### II. O/W cream with Phospholipon^{®} 90 H and octenidine dihydrochloride

| | |
|---|---|
| 6.00% | Phospholipon^{®} 90 H |
| 0.05% | octenidine dihydrochloride |
| 15.00% | octyldodecanol |
| 0.50% | **α**-DL-tocopherol |
| 5.00% | pentylene glycol |
| 73.45% | water, demin. |

a. Heat water to 70°C.
   Add Phospholipon^{®} 90 H and octenidine dihydrochloride and stir in.
   Then homogenize at the highest setting for 30 min.
b. Mix add octyldodecanol, tocopherol and pentylene glycol, homogenizing at intermediate speed.
   Subsequently emulsify at the highest setting for 10 min.
   Stir cream until cold while at intervals homogenizing briefly at intermediate speed.

This formulation was prepared in an IKA L 1000 laboratory reactor with anchor stirrer (= 100 rpm) and Ultra-Turrax T25 (13 500-24 000 rpm).

### III. Hydrogel with Phospholipon^{®} 90 H and octenidine dihydrochloride

| | |
|---|---|
| 6.00% | Phospholipon^{®} 90 H |
| 0.05% | octenidine dihydrochloride |
| 5.00% | propylene glycol |
| 0.20% | sodium carboxyvinyl polymer (Pionier^{®}) NP37G) |
| 88.75% | water, demin. |

### a. Heat water to 70°C.

Add Phospholipon^{®} 90 H and octenidine dihydrochloride and stir in.

Then homogenize at the highest setting for 30 min.

### b. Mix propylene glycol and Pionier^{®} NP37G and add.

Homogenize by stirring until the gel is completely swollen.

This formulation was prepared in an IKA L 1000 laboratory reactor with anchor stirrer (= 100 rpm) and Ultra-Turrax T25 (13 500-24 000 rpm).

### IV. Mixed micelles with Phospholipon^{®} 90 G and octenidine dihydrochloride

| | |
|---|---|
| 4.00% | Phospholipon^{®} 90 H |
| 0.05% | octenidine dihydrochloride |
| 0.30% | sodium chloride |
| 20.00% | polysorbate 20 |
| 2.00% | NaOH solution 10% strength |
| 73.65% | water, demin. |

a. Introduce water into a glass beaker and dissolve sodium chloride therein. Add Phospholipon^{®} 90 G and polysorbate 20 and completely disperse.
b. Add 10% strength NaOH solution and stir until a clear solution results. The pH must be about 10.0. Then add the octenidine dihydrochloride and stir. until dissolution is complete. The mixed micelles are finally filtered (0.2 µm). The pH is about 7-8.

This formulation can be prepared in a glass beaker by a simple stirring technique.

### Example 3: Low cytotoxicity of the preparations according to the invention

The test cells used for in vitro cytotoxicity investigations were mouse fibroblasts of the cell line L929 (ATCC CCL1). Evaluation took place with the aid of two different spectrophotometric methods. With the comparative preparation, 50% of the test cells were not vital after exposure to 32.0 µg/ml (method 1) and 41.3 µg/ml (method 2) for 30 minutes. The preparation according to the invention merely showed a cytotoxicity of less than 20% even at the highest concentration possible in the test, of 250 µg/ml octenidine dihydrochloride.

## Claims

1. Use of liposomes comprising phospholipids for manufacturing an antimicrobial preparation which comprises octenidine dihydrochloride encapsulated in liposomes, for reducing the cytotoxicity of the preparation.

2. Use according to Claim 1, **characterized in that** the preparation is in the form of a solution, dispersion, gel, cream, ointment or suppository.

3. Use according to Claim 1 or Claim 2, **characterized in that** the preparation comprises from 0.001 to 5% by weight, preferably from 0.003 to 1% by weight, more preferably from 0.005 to 0.1% by weight, in particular from 0.01 to 0.06% by weight, octenidine dihydrochloride based on the preparation.

4. Use according to any of the preceding claims, **characterized in that** the liposomes comprise phosphatidylcholine.

5. Use according to Claim 4, **characterized in that** the amount of phospholipid is from 0.1 to 30% by weight, preferably from 0.5 to 20% by weight, in particular from 1 to 10% by weight, for example from 3 to 8% by weight, based on the complete preparation.

6. Process for the manufacture of the antimicrobial preparation according to claim 1, in which water is mixed at elevated temperature with phospholipid and octenidine dihydrochloride, and this mixture is then homogenized.

## Patentansprüche

1. Verwendung von Liposomen, die Phospholipide umfassen, zur Herstellung eines antimikrobiellen Präparats, das in Liposome eingekapseltes Octenidin-Dihydrochlorid zur Verringerung der Zytotoxizität des Präparats umfasst.

2. Verwendung nach Anspruch 1, **dadurch gekennzeichnet, dass** das Präparat in der Form einer Lösung, einer Dispersion, eines Gels, einer Creme, einer Salbe oder eines Suppositoriums ist.

3. Verwendung nach Anspruch 1 oder 2, **dadurch gekennzeichnet, dass** das Präparat 0,001 bis 5 Gew.-%, vorzugsweise 0,003 bis 1 Gew.-%, mehr bevorzugt 0,005 bis 0,1 Gew.-%, insbesondere 0,01 bis 0,06 Gew.-% Octenidin-Dihydrochlorid umfasst, bezogen auf das Präparat.

4. Verwendung nach einem der vorhergehenden Ansprüche, **dadurch gekennzeichnet, dass** die Liposome Phosphatidylcholin umfassen.

5. Verwendung nach Anspruch 4, **dadurch gekennzeichnet, dass** die Phospholipidmenge 0,1 bis 30 Gew.-%, vorzugsweise 0,5 bis 20 Gew.-%, insbesondere 1 bis 10 Gew.-%, beispielsweise 3 bis 8 Gew.-%, beträgt, bezogen auf das komplette Präparat.

6. Verfahren zur Herstellung des antimikrobiellen Präparats nach Anspruch 1, wobei Wasser bei erhöhter Temperatur mit Phospholipid und Octenidin-Dihydrochlorid gemischt wird und dieses Gemisch dann homogenisiert wird.

## Revendications

1. Utilisation de liposomes comprenant des phospholipides pour la fabrication d'une préparation antimicrobienne qui comprend du dichlorhydrate d'octénidine encapsulé dans des liposomes, pour réduire la cytotoxicité de la préparation.

2. Utilisation selon la revendication 1, **caractérisée en ce que** la préparation est sous la forme d'une solution, d'une dispersion, d'un gel, d'une crème, d'un onguent ou d'un suppositoire.

3. Utilisation selon la revendication 1 ou 2, **caractérisée en ce que** la préparation comprend entre 0,001 et 5 % en poids, de préférence entre 0,003 et 1 % en poids, plus préférablement entre 0,005 et 0,1 % en poids, en particulier entre 0,01 et 0,06 % en poids, de dichlorhydrate d'octénidine sur la base de la préparation.

4. Utilisation selon l'une quelconque des revendications précédentes, **caractérisée en ce que** les liposomes comprennent de la phosphatidylcholine.

5. Utilisation selon la revendication 4, **caractérisée en ce que** la quantité de phospholipides est comprise entre 0,1 et 30 % en poids, de préférence entre 0,5 et 20 % en poids, en particulier entre 1 et 10 % en poids, par exemple entre 3 et 8 % en poids, sur la base de la préparation complète.

6. Procédé de fabrication de la préparation antimicrobienne selon la revendication 1, dans lequel de l'eau est mélangée à une température élevée avec des phospholipides et du dichlorhydrate d'octénidine, et ce mélange est ensuite homogénéisé.
